# EUROPEAN PATENT APPLICATION

(11) **EP 2 983 127 A1**
(43) Date of publication of application: **10.02.2016**
(21) Application number: 13880952.0
(22) Date of filing: 01.04.2013
(51) Int. Cl.: G06Q 50/24

(54) **MEDICAL-INFORMATION MANAGEMENT DEVICE, MEDICAL-INFORMATION MANAGEMENT SYSTEM, AND METHOD FOR CONTROLLING MEDICAL-INFORMATION MANAGEMENT DEVICE**

(71) Applicant: Terumo Kabushiki Kaisha, Tokyo 151-0072 (JP)
(72) Inventor: TOUNOOKA Yuuya, Ashigarakami-gun Kanagawa 259-0151 (JP); KATOU Shinji, Ashigarakami-gun Kanagawa 259-0151 (JP)
(74) Representative: Casalonga
(86) International application number: PCT/JP2013/059999
(87) International publication number: WO 2014/162488

(57) **Abstract**

Provided are a medical information management apparatus and the like that can secure objectivity of an analysis of an administration state of a drug, and the like. A medical information management apparatus 10 includes drug administration tendency information 32a for indicating an administration tendency of a plurality of pieces of administration information of a drug used in a medical instrument 2a, and drug administration tendency standard information 34a serving as a standard for judging appropriateness of the administration tendency of the drug administration tendency information, wherein the drug administration tendency standard information includes drug administration amount standard information that is administration amount information of the drug supposed to have a highest administration frequency.

## Description

### Technical Field

The present invention relates to a medical information management apparatus, a medical information management system, and a method of controlling a medical information management apparatus that manage medical information such as drug information used in hospitals and the like.

### Background Art

Conventionally, infusion pumps and the like have been used to appropriately administrate drugs to patients in hospitals and the like (for example, Patent Literature 1).

Databases such as "drug libraries" have been built to manage information such as drug information used in medical instruments such as the infusion pumps, and systems that enable appropriate prescription of the drugs using the infusion pumps and the like have been employed.

Further, in the hospitals, actual administration data and the like of the drugs in the infusion pumps and the like have also been stored in the drug libraries, an administration state thereof has been analyzed, and appropriate administration of the drugs has been performed.

### Citation List

### Patent Literature

Patent Literature 1: JP 2011-87678 A

### Summary of Invention

### Technical Problem

However, it is a person in change who performs the analysis of the administration situation of the drugs, and there is a problem that judgment for the analysis differs depending on each person in charge and appropriate judgment cannot be secured.

Therefore, an objective of the present invention is to provide a medical information management apparatus, a medical information management system, and a method of controlling a medical information management apparatus that can secure objectivity of an analysis of an administration situation of a drug, and the like.

### Solution to Problem

In the present invention, the above-described objective can be achieved by a medical information management apparatus that includes: drug administration tendency information for indicating an administration tendency of a plurality of pieces of administration information of a drug used in a medical instrument; and drug administration tendency standard information serving as a standard for judging appropriateness of the administration tendency of the drug administration tendency information, wherein the drug administration tendency standard information includes drug administration amount standard information that is administration amount information of the drug supposed to have a highest administration frequency.

According to the above-described configuration, the administration tendency of the drug actually used by, for example, an infusion pump or the like that is the medical instrument is illustrated in the drug administration tendency information, for example, a most recent administration rate distribution graph by drug and the like. Further, the administration tendency illustrated in the drug administration tendency information is compared with the drug administration tendency standard information, for example, an administration rate normal distribution graph by drug and the like, so that whether the administration tendency is appropriate can be objectively judged.

That is, an analysis of an administration situation of the drug or the like can be objectively performed.

Further, the drug administration tendency standard information includes, for example, standard administration rate information that is the drug administration amount standard information that is administration amount information of the drug supposed to have a highest administration frequency.

Therefore, the administration amount information in an administration rate range or the like of the drug administration tendency standard information correlated to the standard administration rate information, for example, an administration amount (for example, the number of times of liquid delivery and the like) of a standard administration rate range, and an administration amount (the number of times of liquid delivery or the like) in the administration rate range of the drug administration tendency information correlated to the administration amount information are compared, so that the analysis of an administration situation of the drug or the like can be easily and objectively performed.

Favorably, the medical information management apparatus has a configuration to compare drug specific administration amount information that is the administration amount information of the drug having the highest administration frequency from the drug administration tendency information, and the administration amount information of the drug administration amount standard information, and provide modified recommendation information of the drug administration tendency standard information, when the drug specific administration amount information and the administration amount information of the drug administration amount standard information are judged to be different.

According to the configuration, the drug specific administration amount information that is the administration amount information (the number of times of liquid delivery or the like) having the highest administration frequency from the drug administration tendency information (a most recent administration rate distribution graph by drug or the like), and the administration amount information (the number of times of liquid delivery or the like) of the drug administration amount standard information (standard administration rate information or the like) are compared, and the modified recommendation information of the drug administration tendency standard information is provided when the drug specific administration amount information and the administration amount information (the number of times of liquid delivery or the like) are judged to be different.

That is, by comparison of the administration amount information (the number of times of liquid delivery or the like) of the standard administration rate information or the like serving as a standard, and the actual administration amount information (the number of times of liquid delivery or the like) having the highest administration frequency of the actual drug, whether it is necessary to modify the drug administration tendency standard information (administration rate normal distribution graph by drug or the like) to serve as the standard is judged, and the modified recommendation information of the drug administration tendency standard information (administration rate normal distribution graph by drug or the like) is provided if the modification is necessary.

Therefore, a person in charge or the like who has received the modified recommendation information can promptly modify the information, and the drug administration tendency standard information (administration rate normal distribution graph by drug or the like) that servers as an appropriate standard can be maintained on a constant basis.

Favorably, the medical information management apparatus has a configuration to provide recommendation information used to modify the drug specific administration amount information, as the drug administration amount standard information.

According to the configuration, the recommendation information that enables modification of the drug specific administration amount information that is the administration amount information of the drug actually having the highest administration frequency, as the drug administration amount standard information (standard administration rate information or the like) is provided. Therefore, the person in charge or the like can promptly and easily modify the drug administration tendency standard information (administration rate normal distribution graph by drug or the like).

Favorably, the medical information management apparatus has a justice in which both of the drug specific administration amount information and the drug administration amount standard information are administration amount range information that is range information of an administration amount of a drug, and to judge that the drug specific administration amount information and the drug administration amount standard information are not different, when a difference between the drug specific administration amount information and the drug administration amount standard information is a difference in the administration amount range information within a predetermined allowable range.

According to the configuration, when the difference between the drug specific administration amount information that is the administration amount information of the drug actually having the highest administration frequency, and the drug administration amount standard information (standard administration rate information or the like) falls within the predetermined allowable range (adjacent information or the like) of the administration amount range information (administration rate range or the like), the difference is judged not to be a difference that requires modification of the drug administration tendency standard information (administration rate normal distribution graph by drug or the like).

Therefore, the person in charge or the like is not forced to perform frequent modifications and the like, and an easy-to-use medical information management apparatus can be realized.

Favorably, the medical information management apparatus has a configuration to compare the drug specific administration amount information that is the administration amount information of the drug having the highest administration frequency from the drug administration tendency information, and the drug administration amount standard information, compare the drug administration tendency information and the drug administration tendency standard information (administration rate normal distribution graph by drug, or the like), for each individual administration amount range information divided according to a range of an administration amount of the drug, when the drug specific administration amount information and the drug administration amount standard information are judged to be not different, and provide modified recommendation information of the drug administration tendency standard information, when an accumulated total of differences in the each administration amount range information exceeds a predetermined range.

According to the configuration, even when there is no difference between the drug specific administration amount information that is the administration amount information of the drug having the highest administration frequency, and the drug administration amount standard information (standard administration rate information or the like), the drug administration tendency information of actual use and the drug administration tendency standard information (administration rate normal distribution graph by drug or the like) are compared, for each individual administration amount range information (administration rate range or the like) divided according to the range of the administration amount of the drug. When an accumulation total of differences in the administration amount range information exceeds the predetermined range (50%, for example), the modified recommendation information of the drug administration tendency standard information (administration rate normal distribution graph by drug or the like) is provided.

Therefore, even when there is no difference in the drug specific administration amount information that is the administration amount information of the drug having the highest administration frequency, in the comparison with the drug administration amount standard information (standard administration rate information or the like) that is an index of the modification, when there is a difference between the drug administration tendency information of actual use, and the drug administration tendency standard information (administration rate normal distribution graph by drug or the like), as a whole, modification of the drug administration tendency standard information (administration rate normal distribution graph by drug or the like) is recommended. Therefore, the modified recommendation information can be provided without missing information, if modification is necessary.

Favorably, the medical information management apparatus and a terminal device that is communicatively connected with the medical information management apparatus are included, and the modified recommendation information is configured to be displayed in a display unit of the terminal device.

In the present invention, the above-described objective can be achieved by a method of controlling a medical information management apparatus, the method including: drug administration tendency information for indicating an administration tendency of a plurality of pieces of administration information of a drug used in a medical instrument; and drug administration tendency standard information serving as a standard for judging appropriateness of the administration tendency of the drug administration tendency information, the drug administration tendency standard information including drug administration amount standard information that is specific administration amount information of a drug supposed to have a highest administration frequency, and the method being configured to compare drug specific administration amount information that is the administration amount information of the drug having the highest administration frequency from the drug administration tendency information, and the administration amount information of the drug administration amount standard information, and provide modified recommendation information of the drug administration tendency standard information, when the drug specific administration amount information and the administration amount information of the drug administration amount standard information are judged to be different.

### Advantageous Effects of Invention

As described above, according to the present invention, a medical information management apparatus, a medical information management system, and a method of controlling a medical information management apparatus that can secure objectivity of an analysis of an administration state of a drug, and the like can be provided.

### Brief Description of Drawings

Fig. 1 is a schematic diagram illustrating, for example, a "drug library system" that is a "medical information management system" according to the present invention.
Fig. 2 is a schematic block diagram illustrating principal configurations of a hospital clerical staff terminal illustrated in Fig. 1.
Fig. 3 is a schematic block diagram illustrating principal configurations of a drug library server illustrated in Fig. 1.
Fig. 4 is a schematic block diagram illustrating contents of a "server-side first information storage unit" illustrated in Fig. 3.
Fig. 5 is a schematic block diagram illustrating contents of a "server-side second information storage unit" illustrated in Fig. 3.
Fig. 6 is a schematic block diagram illustrating contents of a "server-side third information storage unit" illustrated in Fig. 3.
Fig. 7 is a schematic flowchart illustrating an operation example and the like of the drug library system according to the present embodiment.
Fig. 8 is another schematic flowchart illustrating an operation example and the like of the drug library system according to the present embodiment.
Fig. 9 is another schematic flowchart illustrating an operation example and the like of the drug library system 1 according to the present embodiment.
Fig. 10 is another schematic flowchart illustrating an operation example and the like of the drug library system according to the present embodiment.
Fig. 11 is a schematic explanatory diagram illustrating a "most recent administration rate distribution graph by drug".
Fig. 12 is a schematic explanatory diagram illustrating an "administration rate normal distribution graph by drug".

### Description of Embodiments

Hereinafter, a favorable embodiment of this invention will be described in detail with reference to the appended drawings, and the like.

Note that the embodiment described below is a mere favorable specific example of the present invention, and thus various technically favorable limitations are added thereto. However, the scope of the present invention is not limited to the embodiment unless there is specific description that limits the present invention.

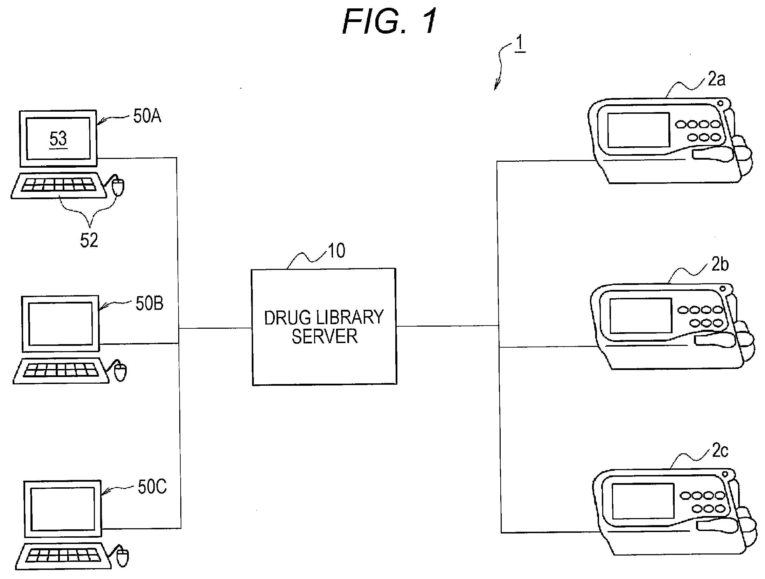

Fig. 1 is a schematic diagram illustrating a "drug library system 1", for example, which is a "medical information management system" according to the present invention.

As illustrated in Fig. 1, the medical information management system 1 includes a "drug library server 10", for example, which is a "medical information management apparatus". Further, for example, hospital clerical staff terminals 50A, 50B, and 50C that are "terminal devices" are communicatively connected with the drug library server 10.

Further, for example, infusion pumps 2a, 2b, and 2c that are "medical instruments" arranged in a hospital are communicatively connected with the drug library server 10. The infusion pump 2a and the like have a configuration to acquire information related to drugs and the like from the drug library server 10.

The drug library server 10 includes a "drug library" related to drugs, that is, detailed data related to prescription of the drugs and the like (for example, drug types (an anticancer drug or an anesthetic), drug names, upper/lower limit values of flow rates (mL/h), upper/lower limit values of injection rates (mL), contraindication information, and the like). This data is mainly transmitted to the infusion pump 2a and the like of Fig. 1, and is used when medical professionals such as nurses and the like who operate the infusion pump 2a and the like administrate the drugs to patients.

Further, information of graphs and the like related to administration situation of respective drugs is also included in the drug library.

Note that the infusion pump 2a and the like are the medical instruments used when the drugs are accurately administrated or the like to the patients, and are arranged to drip devices or the like when highly accurate control or the like is required for the administration of the drugs.

Further, the drug library system 1 has a configuration in which the information of graphs and the like related to administration states of respective drugs included in the drug library can be referred, and the can be modified or the like, by accessing the drug library server 10 from the hospital clerical staff terminal 50A or the like.

The hospital clerical staff terminal 50A and the like illustrated in Fig. 1 include a "hospital clerical staff terminal-side display 53" that is a "display unit" that displays various types of information, and a "hospital clerical staff terminal-side input device 52" for inputting various types of information.

By the way, the drug library server 10, the hospital clerical staff terminal 50A and the like, the infusion pump 2a, and the like illustrated in Fig. 1 include a computer. The computer includes a central processing unit (CPU), a random access memory (RAM), a read only memory (ROM), and the like (those are not illustrated), and these unit and memories are connected through a bus.

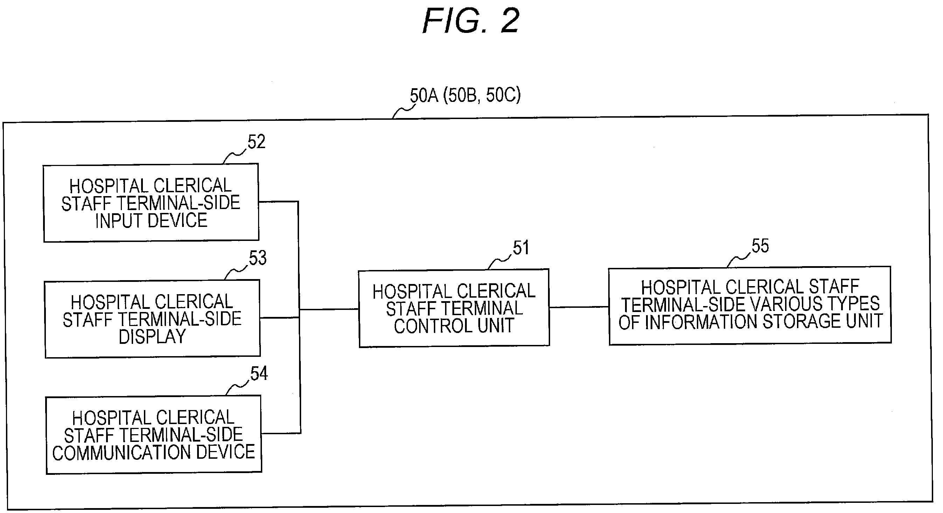

Fig. 2 is a schematic block diagram illustrating principal configurations of the hospital clerical staff terminal 50A and the like illustrated in Fig. 1.

As illustrated in Fig. 2, the hospital clerical staff terminal 50A and the like include a "hospital clerical staff terminal control unit 51".

The "hospital clerical staff terminal control unit 51" has a configuration to control a "hospital clerical staff terminal-side input device 52", a "hospital clerical staff terminal-side display 53", and a "hospital clerical staff terminal-side communication device 54" that performs communication with the drug management server 10 or the like, as illustrated in Fig. 1, and also control a "hospital clerical staff terminal-side various types of information storage unit 55".

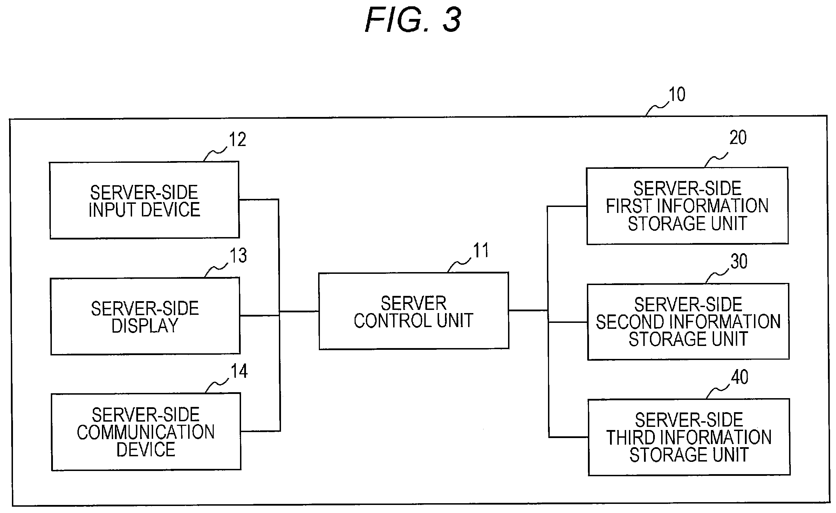

Further, Fig. 3 is a schematic block diagram illustrating principal configurations of the drug library server 10 illustrated in Fig. 1.

As illustrated in Fig. 3, the drug library server 10 includes a "server control unit 11". The server control unit 11 has a configuration to control a "terminal-side input device 12" that inputs data and the like, a "server-side display 13" that displays data and the like, and a "server-side communication device 14" or the like that performs communication with the hospital clerical staff terminal 50A and the like. The server control unit 11 also has a configuration to control a "server-side first information storage unit 20", a "server-side second information storage unit 30" and a "server-side third information storage unit 40".

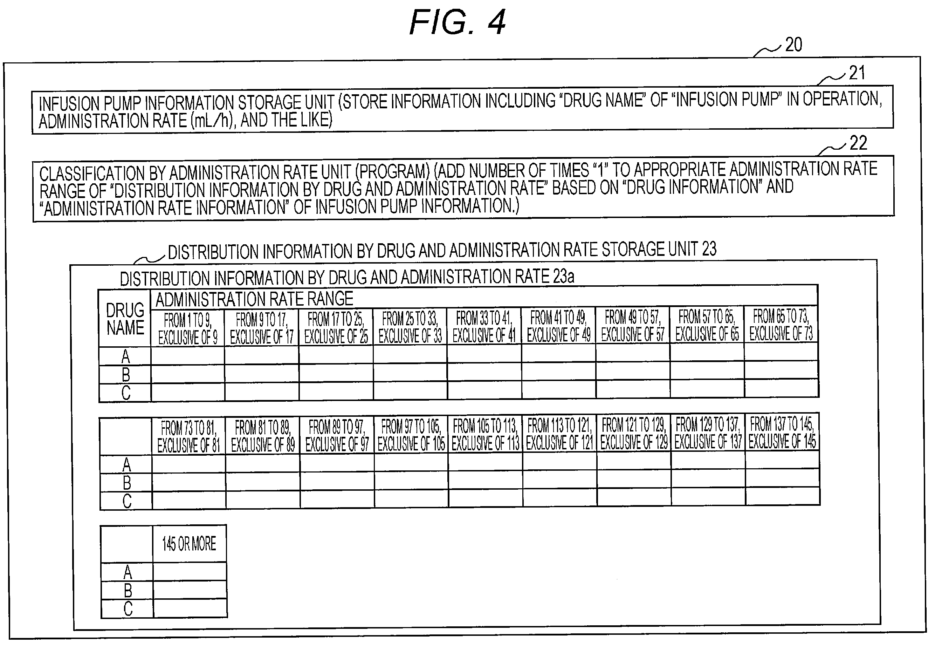

Note that Figs. 4, 5, and 6 are schematic block diagrams respectively illustrating contents of the "server-side first information storage unit 20", the "server-side second information storage unit 30", and the "server-side third information storage unit 40" illustrated in Fig. 3. Respective contents of these blocks will be described below.

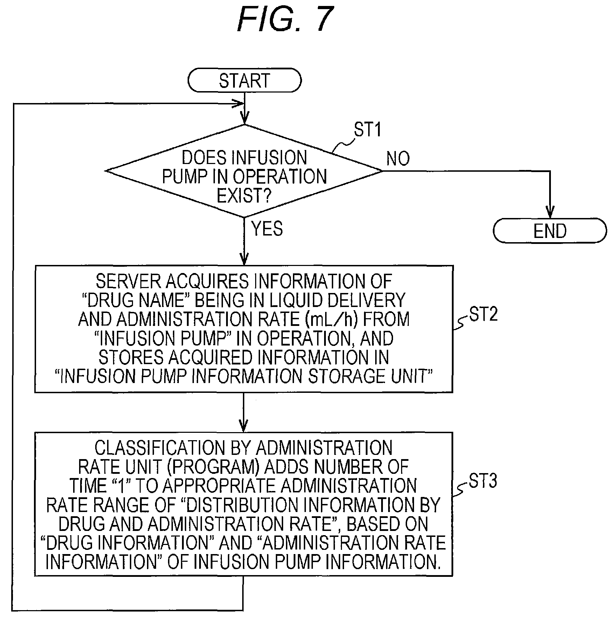

Figs. 7 to 10 are schematic flowcharts illustrating an operation example and the like of the drug library system 1 according to the present embodiment.

In the present embodiment, a user refers to data related to an administration state of a drug stored in the drug library server 10, using the hospital clerical staff terminal 50A or the like. Therefore, in Fig. 7, the drug library server 10 acquires information of the administration situation of the drug from the infusion pump 2a and the like.

First, in step ST (hereinafter, referred to as "ST") 1 of Fig. 7, the drug library server 10 is operated, and whether the infusion pump 2a or the like in operation exists is judged.

In ST1, when the infusion pump 2a or the like in operation exists, the operation proceeds to ST2. In ST2, the drug library server 10 acquires information of a "drug name (for example, a drug A or the like) being in liquid delivery and an administration rate (mL/h) from the "infusion pump 2a or the like" in operation, and stores the acquired information in an "infusion pump information storage unit 21" of Fig. 4.

Next, the operation proceeds to ST3. In ST3, a "classification by administration rate unit (program) 22" of Fig. 4 is operated, and adds the number of times of "1" to an appropriate "administration rate range" of "distribution information by drug and administration rate 23a" of a "distribution information by drug and administration rate storage unit 23" of Fig. 4, based on "drug information (drug A or the like)" and "administration rate information (for example, 8 mL/h or the like)" of infusion pump information of the "infusion pump information storage unit 21".

In the above-described example, the administration rate is "8 mL/h". Therefore, the classification by administration rate unit (program) 22 adds "1" to the "administration rate range" of "from 1 to 9, exclusive of 9" of the "distribution information by drug and administration rate 23a", as the number of times of liquid delivery.

This "administration rate range" is an example of "administration amount information" and "administration amount range information".

In the above operation, the data of the number of times of liquid livery of the infusion pump 2a or the like of each drug and each administration rate range is stored in the "distribution information by drug and administration rate storage unit 23" of Fig. 4.

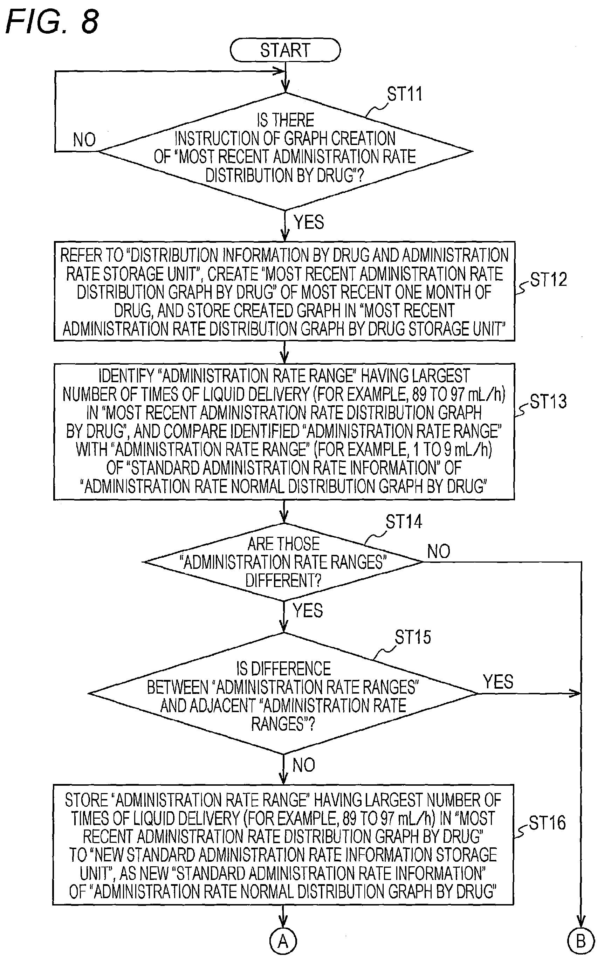

Next, a process in which a person in change accesses the drug library server 10 through the hospital clerical staff terminal 50A or the like of Fig. 1, and acquires information for analyzing an administration state of each drug (drug A or the like) will be described using Figs. 8 to 10.

First, in ST11 of Fig. 8, the drug library server 10 judges whether there is an instruction of execution of an analysis process of an administration situation of each drug, from the hospital clerical staff terminal 50A or the like, to be specific, whether there is an instruction of creation of a graph of "most recent administration rate distribution by drug".

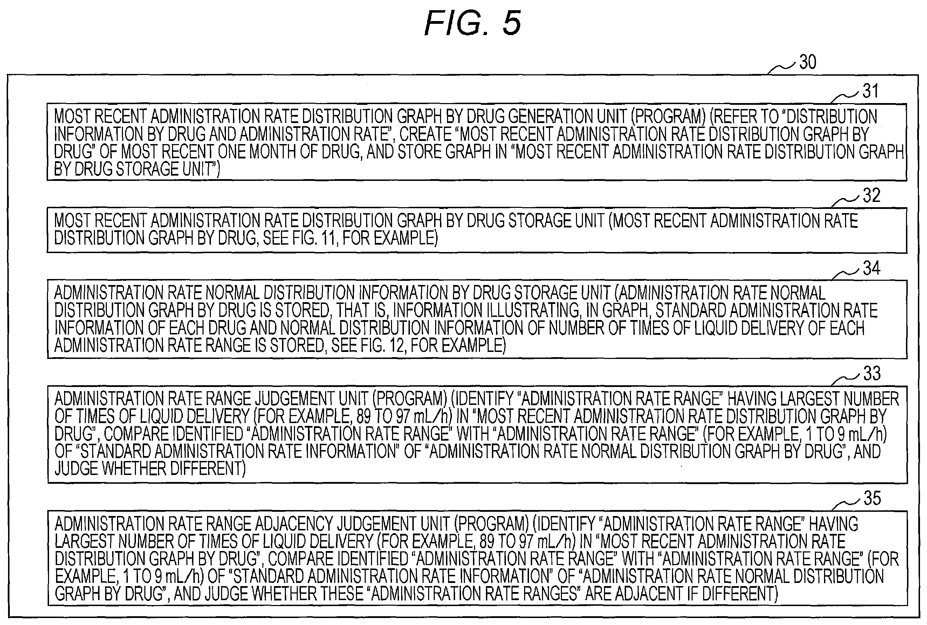

In ST11, when the drug library server 10 judges that there has been the instruction of creation of a graph of "most recent administration rate distribution by drug", the process proceeds to ST12. In ST12, a "most recent administration rate distribution graph by drug generation unit (program) 31" of Fig. 5 is operated, and refers to the "distribution information by drug and administration rate 23a" of Fig. 4, creates a "most recent administration rate distribution graph by drug 32a" of a most recent one month of an appropriate drug (for example, the drug A or the like), and stores the created graph in a "most recent administration rate distribution graph by drug storage unit 32" of Fig. 5.

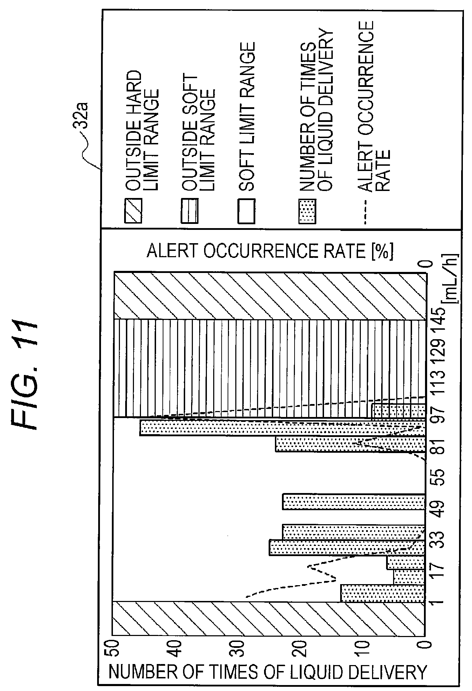

Fig. 11 is a schematic explanatory diagram illustrating the "most recent administration rate distribution graph by drug 32a". As illustrated in Fig. 11, in the "most recent administration rate distribution graph by drug 32a", the "administration rate ranges" are formed on a horizontal axis such that the administration rate of the drug is divided every 8 mL/h. Then, the number of times of liquid delivery is illustrated on a vertical axis by a bar graph for each administration rate range.

Further, in the "most recent administration rate distribution graph by drug 32a" of Fig. 11, information of "hard limit", "soft limit", and the like, which is administration limitation information of the drug, is also displayed.

Note that the "most recent administration rate distribution graph by drug 32a" is an example of "drug administration tendency information".

Next, the operation proceeds to ST13. In ST13, an "administration rate range judgment unit (program) 33" of Fig. 5 is operated, and identifies the "administration rate range" having the largest number of times of liquid delivery in the "most recent administration rate distribution graph by drug 32a" of Fig. 11, for example, 89 to 97 mL/h. This "administration rate range" having the largest number of times of liquid delivery is an example of "drug specific administration amount information".

Next, the operation refers to an "administration rate normal distribution graph by drug 34a" stored in an "administration rate normal distribution information by drug storage unit 34" of Fig. 5.

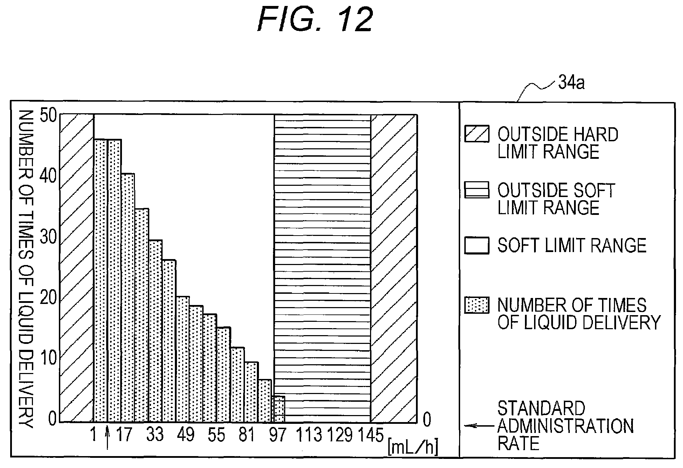

Fig. 12 is a schematic explanatory diagram illustrating the "administration rate normal distribution graph by drug 34a".

As illustrated in Fig. 12, in the "administration rate normal distribution graph by drug 34a", the "administration rate ranges" are formed on the horizontal axis such that the administration rate is divided every 8 mL/h, similarly to the "most recent administration rate distribution graph by drug 32a" of Fig. 11. Then, the number of times of liquid delivery is illustrated on the vertical axis by a bar graph for each administration rate range.

The number of times of liquid delivery of each administration rate range forms distribution of the number of liquid delivery that is supposed to be or is expected or the like to distribute as such. In the case of Fig. 12, the number of times of liquid delivery in the administration rate range of 1 to 9 mL/H is largest, and the distribution is formed such that the number of times of liquid delivery becomes smaller as the administration rate becomes larger.

That is, when distribution of the number of times of liquid delivery of the drug is close to such distribution, the person in charge or the like can judge that the administration situation of the drug is close to the normal distribution, and is appropriate. Therefore, the distribution serves as standard data of the analysis.

In other words, in the "administration rate normal distribution graph by drug 34a", standard administration rate information, and information of normal distribution information of the number of times of liquid delivery of each administration rate range illustrated by a graph, of each drug, are stored.

Further, in the "administration rate normal distribution graph by drug 34a", a "standard administration rate (an example of "drug administration amount standard information") that is the "administration rate range" expected to have a maximum accumulation total of the number of times of liquid delivery of the drug is also illustrated. In the case of Fig. 12, the "administration rate range" of "from 1 to 9 mL/h, exclusive of 9" falling into the standard administration rate is displayed.

That is, the "standard administration rate" is the "administration rate range" supposed to have a highest administration frequency. As described above, the "administration rate normal distribution graph by drug 34a" is as an example of "drug administration tendency standard information".

In ST13, the "administration rate range" having the largest number of times of liquid delivery in the "most recent administration rate distribution graph by drug 32a" of Fig. 11, for example, "89 to 97 mL/h", and the "administration rate range" (for example, 1 to 9 mL/h) of the "standard administration rate information" in the "administration rate normal distribution graph by drug 34a" are compared.

Next, the process proceeds to ST14. In ST14, whether the "administration rate range (for example, "89 to 97 mL/h")" in the "most recent administration rate distribution graph by drug 32a", and the "administration rate range (for example, 1 to 8 mL/h)" of the "standard administration rate information" in the "administration rate normal distribution graph by drug 34a" are different is judged.

In the present embodiment, these administration rate ranges are different, and thus the judgment is "YES".

As described above, in the present embodiment, the person in charge or the like analyzes the administration situation of the drug. To be specific, the person in charge or the like compares the "administration rate normal distribution graph by drug 34a" that is the normal distribution serving as a standard, and the "most recent administration rate distribution graph by drug 32a" that is an actual administration record, and judges whether the administration state is appropriate. Further, the present embodiment can provide an objective and easy standard to the person in charge or the like who analyzes the administration state of the drug.

However, existence of the difference in the "administration rate range" of the "standard administration rate information", which is the most important index, indicates necessity of modification or the like of the "administration rate normal distribution graph by drug 34a".

Therefore, the present embodiment has a configuration to be able to provide information about, for example, whether the "administration rate normal distribution graph by drug 34a" needs to be modified or the like, to the person in charge or the like.

Next, the process proceeds to ST15. In ST15, an "administration rate range adjacency judgment unit (program) 35" is operated, and judges whether the different "administration rate range (for example, "89 to 97 mL/h")" in the "most recent administration rate distribution graph by drug 32a" and "administration rate range (for example, 1 to 9 mL/h)" of the "standard administration rate information" in the "administration rate normal distribution graph by drug 34a" are adjacent "administration rate ranges".

That is, the "administration rate ranges" adjacent to the administration rate range of "89 to 97 mL/h" are "81 to 89 mL/h" and "97 to 105 mL/h", for example, as illustrated in the "distribution information by drug and administration rate 23a" of Fig. 4. If the difference is in these adjacent "administration rate ranges", the difference is judged to be a minor difference that does not require the modification or the like of the "administration rate normal distribution graph by drug 34a" (the difference falls within an allowable range).

Therefore, the person in charge or the like is not forced to perform frequent modification or the like of the "administration rate normal distribution graph by drug 34a" due to occurrence of the minor difference and the like, and an easy-to-use drug library system 1 can be realized.

In the above-described example of the present embodiment, the different "administration rate range (for example, "89 to 97 mL/h")" in the "most recent administration rate distribution graph by drug 32a" and "administration rate range (for example, 1 to 8 mL/h)" of the "standard administration rate information" in the "administration rate normal distribution graph by drug 34a" are not adjacent "administration rate ranges", and are substantially different. Therefore, in ST15, the judgment is "NO", and the process proceeds to ST16.

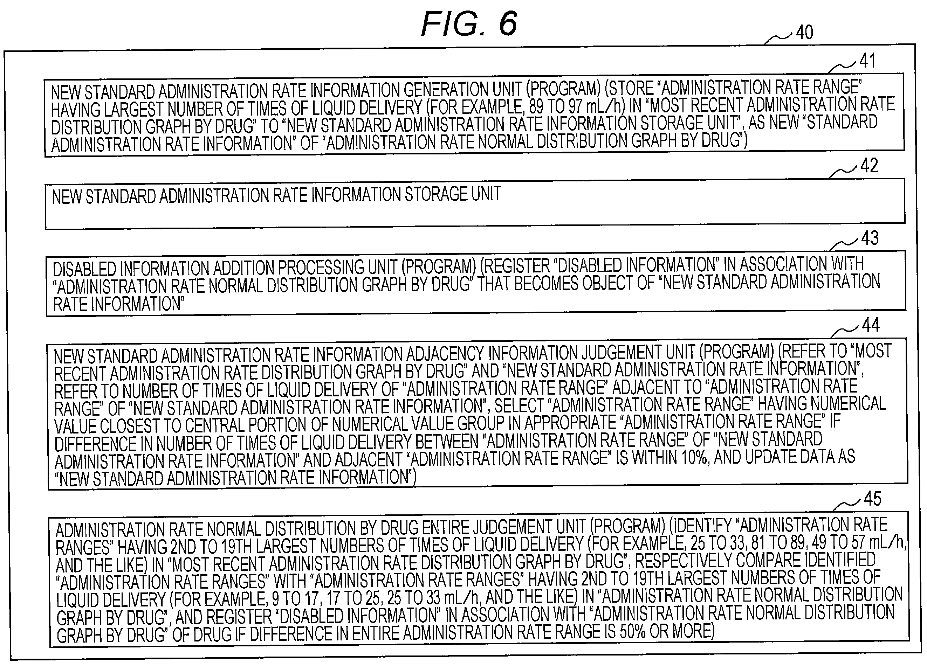

In ST16, a "new standard administration rate information generation unit (program) 41" is operated, and stores the "administration rate range" having the largest number of times of liquid delivery (for example, 89 to 97 mL/h) in the "most recent administration rate distribution graph by drug 32a" of Fig. 11, to a "new standard administration rate information storage unit 42" of Fig. 6, as new "standard administration rate information" of the "administration rate normal distribution graph by drug 34a" of Fig. 12.

That is, the "administration rate range" of "89 to 97 mL/h" is stored as desirable "standard administration rate information".

Next, the process proceeds to ST17. In ST17, a "disabled information addition processing unit (program) 43" of Fig. 6 is operated, and stores the "administration rate normal distribution graph by drug 34a" of Fig. 12 that has been the object of the "new standard administration rate information" stored in the "new standard administration rate information storage unit 42" in ST16, in association with "disabled information", to the "administration rate normal distribution information by drug storage unit 34" of Fig. 4.

Next, the process proceeds to ST18. In ST18, a "new standard administration rate information adjacency information judgment unit (program) 44" is operated, and refers to the "most recent administration rate distribution graph by drug 32a" of Fig. 11 and the "new standard administration rate information storage unit 42" of Fig 6, and refers to the number of times of liquid delivery of the "administration rate range" adjacent to the "administration rate range (for example, "89 to 97 mL/h")" of the "new standard administration rate information".

In the case of Fig. 11, the "administration rate ranges" adjacent to "89 to 97 mL/h" are "81 to 89 mL/h" and "97 to 105 mL/h", and the numbers of times of liquid delivery in these administration rate ranges are about 25 times and 8 times.

Next, the process proceeds to ST19. In ST19, whether differences between the numbers of times of liquid delivery (for example, 25 times and 8 times) of the adjacent "administration rate ranges" and the number of times of liquid delivery (for example, about 47 times) of the "new standard administration rate information", for example, "89 to 97 mL/h" of the "new standard administration rate information storage unit 42" of Fig. 6 are within 10% is judged.

In the case of the above-described example of the present embodiment, the numbers of times of liquid delivery of the adjacent administration rate ranges are 25 times and 8 times, and the differences from 47 times that is the number of times of liquid delivery of "89 to 97 mL/h" are not within 10%. Therefore, the judgment in ST19 is "NO".

However, for example, when the numbers of times of liquid delivery of the "administration rate ranges" adjacent to the number of times of liquid delivery (for example 47 times) of "89 to 97 mL/h" are 46 times and 45 times, and the differences from the 47 times are within 10%, the process proceeds to ST20.

In ST20, in the "administration rate range (89 to 97 mL/h)", the "administration rate range" having a numerical value closest to a central portion of a numerical value group is selected.

That is, in the case of the "most recent administration rate distribution graph by drug 32a" of Fig. 11, numerical values are "1 to 145 mL/h", and the central portion is "72.5 mL/h".

Therefore, the numerical value of the "administration rate range (89 to 97 mL/h)" close to "72.5 mL/h", that is, "81 to 89 mL/h" is selected.

Then, the "administration rate range" of "81 to 89 mL/h" is updated and registered to the "new standard administration rate information storage unit 42", as the "new standard administration rate information" of Fig. 6.

As described above, the "new standard administration rate information" is defined to a position close to the central portion of the numerical value group, so that the nurse or the like can easily perform a setting operation when setting the administration rate to the drug in the infusion pump 2a or the like.

To be specific, usually, when setting a numerical value with a dial of the infusion pump 2a or the like, the numerical value of initial setting is often the numerical value of the central portion. Therefore, setting to a numerical value of the "administration rate range" that is close to the numerical value of the central portion can be easily operated by an operator such as the nurse or the like.

Next, the process proceeds to ST21. In ST21, disablement processing having been applied to the "administration rate normal distribution graph by drug 34a" of the drug, and a request of review of the disabled graph are displayed on the "hospital clerical staff terminal-side display 53" of Fig. 1. Further, recommendation of the "administration rate range" of the "new standard administration rate information" of the "new standard administration rate information storage unit 42" of Fig. 6, as the "standard administration rate information", is displayed. These display contents are examples of "recommendation information".

Therefore, the person in charge or the like who analyzes or the like the administration state of the drug can promptly learn necessity of modification or the like of the "administration rate normal distribution graph by drug 34a" that is to be used in the own analysis. Further, the "standard administration rate information" that should be used is displayed together in the modification. Therefore, the person in charge can easily perform modification work.

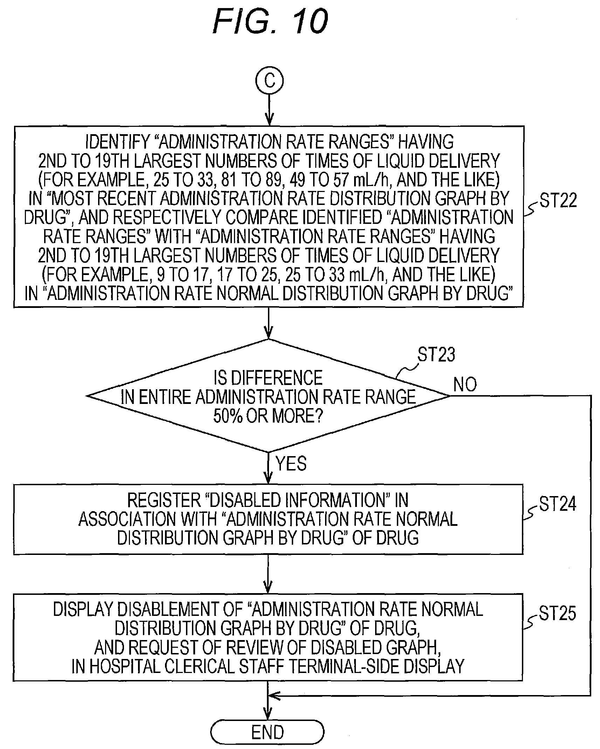

Meanwhile, in ST14 of Fig. 8, when the "administration rate range (for example, "89 to 97 mL/h")" in the "most recent administration rate distribution graph by drug 32a" and the "administration rate range" of the "standard administration rate information" in the "administration rate normal distribution graph by drug 34a" are not different, the process proceeds to ST22 of Fig. 10.

In ST14, even when the "administration rate range" in the "most recent administration rate distribution graph by drug 32a" and the "administration rate range" of the "standard administration rate information" in the "administration rate normal distribution graph by drug 34a" are not different, there is a case where the person in charge or the like should not use the data of the "administration rate normal distribution graph by drug 34a", as the analysis standard of the administration state of the drug.

Therefore, in the present embodiment, in ST22 and subsequent steps, whether the data of the "administration rate normal distribution graph by drug 34a" should be used as the analysis standard of the administration state of the drug is judged.

First, in ST22, an "administration rate normal distribution by drug entire judgment unit (program) 45" of Fig. 6 is operated, and identifies the "administration rate ranges" having 2nd to 19th largest numbers of times of liquid delivery (for example, 25 to 33, 81 to 89, 49 to 57 mL/h, and the like) in the "most recent administration rate distribution graph by drug 32a" of Fig. 11, and compares the identified administration rate ranges with the "administration rate ranges" having 2nd to 19th largest numbers of times of liquid delivery (for example, 9 to 17, 17 to 25, 25 to 33 mL/h, and the like) in the "administration rate normal distribution graph by drug 34a" of Fig. 12, respectively.

That is, the "administration rate ranges" other than the "standard administration rate information", regarding the "most recent administration rate distribution graph by drug 32a" and the "administration rate normal distribution graph by drug 34a", are compared.

These "administration rate ranges" are examples of the "administration amount range information".

Next, the process proceeds to ST23. In ST23, whether the entire difference of the "administration rate ranges" falls within a "predetermined range", for example, within 50% or more is judged.

That is, descending orders of the numbers of times of liquid delivery of the "administration rate ranges" of the "most recent administration rate distribution graph by drug 32a" and the "administration rate normal distribution graph by drug 34a" are compared, for each "administration rate range", for example, "25 to 33 mL/h", and whether the descending orders of the "administration rate ranges" are different is judged. Further, whether the difference in the "administration rate ranges" is 50% (for example, 9 "administration rate ranges") or more, of the entire "administration rate ranges" (for example, 18 "administration rate ranges"), is judged.

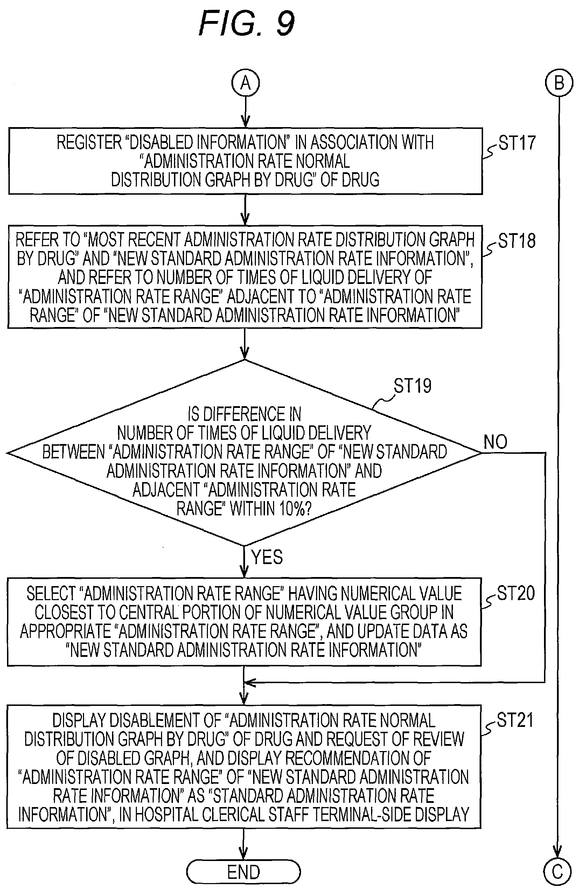

In ST23, when the difference is judged to be 50% or more, the process proceeds to ST24. In ST24, similarly to ST17 of Fig. 9, the "administration rate normal distribution graph by drug 34a" of the drug is registered to the "administration rate normal distribution by drug storage unit 34", in association with the "disabled information".

As described above, in the present embodiment, in ST14, a drawback of when the appropriateness of the "administration rate normal distribution graph by drug 34a" is judged with the "administration rate ranges" of the "standard administration rate information" is compensated, and the appropriateness as a whole is also judged. Therefore, judgment of appropriateness without omission can be performed.

Next, the process proceeds to ST25. In ST25, the disablement processing having been applied to the "administration rate normal distribution graph by drug 34a" of the drug, and a request of review of the disabled graph are displayed on the "hospital clerical staff terminal-side display 53".

Therefore, the person in charge or the like who analyzes or the like the administration situation of the drug can promptly learn necessity of modification or the like of the "administration rate normal distribution graph by drug 34a" that is to be used in the own analysis. Therefore, an extremely easy-to-use system can be realized.

By the way, the present invention is not limited to the above-described embodiment. In the present embodiment, description has been given using the infusion pump 2a or the like as an example of the medical instrument. However, the present invention is not limited to the example, and can be favorably applied to other medical instruments or the like, for example, a syringe pump.

### Reference Signs List

- 1: Drug library system
- 2a, 2b, 2c: Infusion pump
- 10: Drug library server
- 11: Server control unit
- 12: Terminal-side input device
- 13: Server-side display
- 14: Server-side communication device
- 20: Server-side first information storage unit
- 21: Infusion pump information storage unit
- 22: Classification by administration rate unit (program)
- 23: Distribution information by drug and administration rate storage unit
- 23a: Distribution information by drug and administration rate
- 30: Server-side second information storage unit
- 31: Most recent administration rate distribution graph by drug generation unit (program)
- 32: Most recent administration rate distribution graph by drug storage unit
- 32a: Most recent administration rate distribution graph by drug
- 33: Administration rate range judgment unit (program)
- 34: Administration rate normal distribution information by drug storage unit
- 34a: Administration rate normal distribution graph by drug
- 35: Administration rate range adjacency judgment unit (program)
- 40: Server-side third information storage unit
- 41: New standard administration rate information generation unit (program)
- 42: New standard administration rate information storage unit
- 43: Disabled information addition processing unit (program)
- 44: New standard administration rate information adjacency information judgment unit (program)
- 45: Administration rate normal distribution by drug entire judgment unit (program)
- 50A, 50B, 50C: Hospital clerical staff terminal
- 51: Hospital clerical staff terminal control unit
- 52: Hospital clerical staff terminal-side input device
- 53: Hospital clerical staff terminal-side display
- 54: Hospital clerical staff terminal-side communication device
- 55: Hospital clerical staff terminal-side various types of information storage unit

## Claims

1. A medical information management apparatus comprising:
drug administration tendency information for indicating an administration tendency of a plurality of pieces of administration information of a drug used in a medical instrument; and
drug administration tendency standard information serving as a standard for judging appropriateness of the administration tendency of the drug administration tendency information, wherein
the drug administration tendency standard information includes drug administration amount standard information that is administration amount information of the drug supposed to have a highest administration frequency.

2. The medical information management apparatus according to claim 1, having a configuration to compare drug specific administration amount information that is the administration amount information of the drug having the highest administration frequency from the drug administration tendency information, and the administration amount information of the drug administration amount standard information, and provide modified recommendation information of the drug administration tendency standard information, when the drug specific administration amount information and the administration amount information of the drug administration amount standard information are judged to be different.

3. The medical information management apparatus according to claim 2, having a configuration to provide recommendation information used to modify the drug specific administration amount information, as the drug administration amount standard information.

4. The medical information management apparatus according to claim 2 or 3, having justice
in which both of the drug specific administration amount information and the drug administration amount standard information are administration amount range information that is range information of an administration amount of a drug, and
to judge that the drug specific administration amount information and the drug administration amount standard information are not different, when a difference between the drug specific administration amount information and the drug administration amount standard information is a difference in the administration amount range information within a predetermined allowable range.

5. The medical information management apparatus according to any one of claims 1 to 4, having a configuration to compare the drug specific administration amount information that is the administration amount information of the drug having the highest administration frequency from the drug administration tendency information, and the drug administration amount standard information, compare the drug administration tendency information and the drug administration tendency standard information (administration rate normal distribution graph by drug, or the like), for each individual administration amount range information divided according to a range of an administration amount of the drug, when the drug specific administration amount information and the drug administration amount standard information are judged to be not different, and provide modified recommendation information of the drug administration tendency standard information, when an accumulated total of differences in the each administration amount range information exceeds a predetermined range.

6. A medical information management system comprising:
the medical information management apparatus according to any one of claims 2 to 5; and
a terminal device configured to communicatively connected with the medical information management apparatus, wherein
the modified recommendation information is configured to be displayed in a display unit (hospital clerical staff terminal-side display) of the terminal device.

7. A method of controlling a medical information management apparatus, the method comprising:
drug administration tendency information for indicating an administration tendency of a plurality of pieces of administration information of a drug used in a medical instrument; and
drug administration tendency standard information serving as a standard for judging appropriateness of the administration tendency of the drug administration tendency information,
the drug administration tendency standard information including drug administration amount standard information that is specific administration amount information of a drug supposed to have a highest administration frequency, and
the method being configured to compare drug specific administration amount information that is the administration amount information of the drug having the highest administration frequency from the drug administration tendency information, and the administration amount information of the drug administration amount standard information, and provide modified recommendation information of the drug administration tendency standard information, when the drug specific administration amount information and the administration amount information of the drug administration amount standard information are judged to be different.
